(19) Europäisches Patentamt
European Patent Office
Office européen des brevets

(11) **EP 3 899 527 B1**

(12) **FASCICULE DE BREVET EUROPEEN**

(45) Date de publication et mention de la délivrance du brevet:
**14.12.2022 Bulletin 2022/50**

(21) Numéro de dépôt: **19842834.4**

(22) Date de dépôt: **17.12.2019**

(51) Classification Internationale des Brevets (IPC):
**G01N 33/00** *(2006.01)*

(52) Classification Coopérative des Brevets (CPC):
**G01N 33/0006; G01N 33/0075**

(86) Numéro de dépôt international:
**PCT/FR2019/053130**

(87) Numéro de publication internationale:
**WO 2020/128311 (25.06.2020 Gazette 2020/26)**

(54) **PROCÉDÉ DE CALIBRATION D'UN CAPTEUR DE GAZ ET DISPOSITIF ASSOCIÉ**

VERFAHREN ZUR KALIBRIERUNG EINES GASSENSORS UND ZUGEHÖRIGE VORRICHTUNG

METHOD FOR CALIBRATING A GAS SENSOR AND ASSOCIATED DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.12.2018 FR 1873407**

(43) Date de publication de la demande:
**27.10.2021 Bulletin 2021/43**

(73) Titulaire: **Elichens**
**38040 Grenoble (FR)**

(72) Inventeur: **LEBEGUE, Benjamin**
**38000 Grenoble (FR)**

(74) Mandataire: **INNOV-GROUP**
**310, avenue Berthelot**
**69372 Lyon Cedex 08 (FR)**

(56) Documents cités:
**US-A1- 2018 136 180     US-A1- 2018 156 766**

- **HASENFRATZ DAVID ET AL: "On-the-Fly Calibration of Low-Cost Gas Sensors", 15 février 2012 (2012-02-15), INTELLIGENT VIRTUAL AGENT. IVA 2015. LNCS; [LECTURE NOTES IN COMPUTER SCIENCE; LECT.NOTES COMPUTER], SPRINGER, BERLIN, HEIDELBERG, PAGE(S) 228 - 244, XP047371318, ISBN: 978-3-642-17318-9 1. Introduction 4. Multi-hop Calibration 5.1 Experimental Results**

**Description**

**DOMAINE TECHNIQUE**

Le domaine technique de l'invention est la calibration d'un capteur de gaz destiné à réaliser des mesures de gaz dans l'environnement, et en particulier en environnement urbain ou périurbain.

**ART ANTERIEUR**

L'obtention de cartographies décrivant la distribution spatiale de concentrations en molécules ou en particules nocives est un besoin répondant à une attente de la population et des autorités, en particulier dans des zones géographiques sensibles, telles des zones urbaines ou plus généralement dans des zones susceptibles d'être affectées par la pollution atmosphérique. De nombreux modèles ont été développés, permettant d'établir des cartographies de pollution atmosphérique et de prévoir leurs évolutions temporelles. Ces modèles sont alimentés par des capteurs répartis dans les zones géographiques examinées.

A partir de données relatives aux sources d'émissions de polluants, et en considérant des paramètres liés aux conditions topographiques ou météorologiques, les modèles permettent d'établir la distribution spatiale de concentrations de molécules ou de particules polluantes dans l'environnement, ce dernier faisant l'objet d'un maillage spatial.

Des agences régionales ou nationales exploitent des stations de mesure réparties sur le territoire, dites stations de référence, qui permettent d'obtenir des mesures régulières de la concentration de polluants atmosphériques. Ces derniers sont par exemple $NO_2$, $O_3$, CO, ou encore des particules fines, par exemple des particules de diamètre inférieur ou égal à 10 $\mu$m (PM 10) ou des particules de diamètre inférieur à 2.5 $\mu$m (PM 2.5). Les mesures réalisées par certaines agences sont ouvertes, c'est-à-dire aisément accessibles au public. Au niveau européen, il est par exemple possible d'obtenir les concentrations de ces polluants sur le site internet de l'agence européenne de l'environnement. En France, des agences régionales gèrent des stations de mesure, ce qui permet d'obtenir des cartographies de polluants ainsi que des prévisions. Les stations de mesures sont des dispositifs fiables, mais onéreux et encombrants. De ce fait, il est difficile d'envisager leur déploiement selon un maillage spatial fin. Leur nombre se limite à quelques unités par agglomération, voire 10 à 20 pour les agglomérations les plus importantes.

Or, pour obtenir une cartographie plus précise, et tenant compte de particularités locales, par exemple un trafic localement encombré, il est préférable de déployer un grand nombre de capteurs de mesures, ces derniers étant espacés de quelques centaines de mètres seulement. Cela permet d'obtenir des cartographies plus réactives à la survenue de singularités locales, influant sur les concentrations de polluants. Le document WO2018178561 décrit par exemple un procédé de réalisation d'une cartographie dans l'environnement à partir de capteurs répartis selon un maillage dense. Compte tenu du nombre de capteurs utilisés, ces derniers ont une conception plus simple, et un coût moindre que les stations de mesures précédemment décrites. En contrepartie, il faut veiller à ce que les données mesurées soient fiables, de façon à obtenir des cartographies les plus exactes possibles.

Une façon de vérifier l'exactitude des mesures délivrées par des capteurs est de les exposer à des concentrations connues de gaz. Mais ce type de calibration est difficilement réalisable sur le terrain, et impose donc que les capteurs testés soient déplacés dans un laboratoire, puis exposés à un gaz étalon, avant d'être déployés à nouveau sur le terrain. On conçoit que ce type de calibration n'est pas envisageable lorsque le nombre de capteurs dépasse plusieurs dizaines, ou plusieurs centaines d'unités.

Les documents US2018/136180 A1 et US2018/156766 A1 représentent l'art antérieur pertinent.

L'invention adresse ce problème, en proposant un procédé simple pour vérifier la qualité des données mesurées par les capteurs et, si besoin, procéder à leur calibration, tout en maintenant les capteurs déployés sur le terrain, et sans manipulation de ces derniers.

**EXPOSE DE L'INVENTION**

Un premier objet de l'invention est un procédé de calibration d'un capteur de gaz, selon la revendication 1, le capteur de gaz appartenant à un réseau de capteurs répartis en différentes positions dans une zone géographique, le capteur de gaz étant destiné à mesurer une concentration d'un analyte dans l'air, la zone géographique comportant au moins une station de référence, distante du capteur de gaz, la station de référence étant destinée à mesurer une concentration d'analyte dans l'air, le procédé comportant les étapes suivantes :

    a) association d'au moins une station de référence au capteur de gaz;
    b) durant une plage temporelle de calibration, mesure d'une concentration d'analyte par le capteur de gaz et prise en compte d'une concentration d'analyte mesurée par chaque station de référence associée au capteur de gaz ;
    c) à partir de la mesure de la concentration d'analyte mesurée par chaque station de référence lors de la plage temporelle de calibration, estimation d'une concentration d'analyte à la position du capteur de gaz;
    d) comparaison entre la concentration d'analyte estimée lors de l'étape c) et la concentration d'analyte mesurée par le capteur de gaz lors de l'étape b) ;
    e) en fonction de la comparaison effectuée lors de l'étape d), calibration du capteur de gaz.

La concentration peut être exprimée, par exemple, en quantité par unité de volume, ou en masse par unité de volume.

Selon un mode de réalisation, le procédé comporte, préalablement aux étapes a) à e), une étape d'apprentissage de façon à :

- sélectionner une station de référence ou plusieurs stations de référence, parmi une pluralité de stations de référence, de telle sorte que durant la plage temporelle de calibration, la concentration d'analyte mesurée par chaque station de référence sélectionnée est corrélée à la concentration d'analyte à la position du capteur de gaz:;
- déterminer un estimateur de la concentration d'analyte à la position du capteur de gaz à partir de la concentration d'analyte mesurée par chaque station de référence sélectionnée ;

de telle sorte que:

- lors de l'étape a), chaque station de référence sélectionnée lors de l'étape d'apprentissage est associée au capteur de gaz ;
- lors de l'étape c), l'estimation de la concentration d'analyte à la position du capteur de gaz est effectuée en appliquant l'estimateur déterminé lors de l'étape d'apprentissage.

La plage temporelle de calibration peut être déterminée lors de l'étape d'apprentissage. L'étape d'apprentissage peut mettre en œuvre un réseau de neurones, de façon à sélectionner au moins une station de référence et établir l'estimateur et éventuellement la plage temporelle de calibration.

Selon l'invention :

- l'analyte est émis dans la zone géographique selon une concentration variable au cours d'une période temporelle prédéterminée, par exemple une journée, la concentration d'analyte émise variant entre un minimum et un maximum au cours de chaque période temporelle prédéterminée, par exemple au cours de chaque journée ;
- la plage temporelle de calibration est déterminée de façon à correspondre à une émission minimale de l'analyte dans la zone géographique considérée, durant chaque période temporelle considérée, par exemple durant chaque journée.

Lors de l'étape c), la concentration d'analyte à la position du capteur de gaz peut être :

- considérée comme égale à la concentration d'analyte mesurée par une station de référence associée au capteur de gaz ;

- ou estimée en appliquant un modèle de propagation, à partir de la concentration d'analyte mesurée par au moins une station de référence associée au capteur de gaz.

L'analyte peut être émis par des moyens de transport ou de chauffage ; la plage temporelle de calibration est alors comprise entre minuit et 6 heures du matin, et de préférence entre 3 heures du matin et 6 heures du matin.

Un autre objet de l'invention est un dispositif d'estimation d'une concentration d'un analyte dans une zone géographique, le dispositif comportant :

- plusieurs capteurs de gaz répartis dans la zone géographique, en différentes positions, chaque capteur de gaz étant configuré pour mesurer une concentration d'analyte à différents instants de mesure,
- une unité de traitement, recevant les mesures d'au moins un capteur de gaz ;

le dispositif étant caractérisé en ce que l'unité de traitement est configurée pour mettre en œuvre un procédé selon le premier objet de l'invention, en utilisant au moins une station de mesure de référence située dans la zone géographique.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1 schématise une implantation de capteurs élémentaires de gaz formant un réseau de capteurs couvrant une zone géographique d'intérêt, dans laquelle se trouvent également des stations de référence.

La figure 2A illustre la variabilité diurne des concentrations d'un polluant en fonction de la position géographique des capteurs de gaz.

La figure 2B illustre la variabilité nocturne des concentrations d'un polluant en fonction de la position géographique des capteurs de gaz.

La figure 3A montre un profil journalier moyen, durant une durée de 7 mois, de différences de concentrations horaires entre deux stations de référence.

La figure 3B montre un profil journalier moyen, durant une durée de 7 mois, de différences de concentrations horaires entre deux stations de référence.

La figure 3C montre un profil journalier moyen, durant une durée de 7 mois, de différences de concentrations horaires entre deux stations de référence.

La figure 3D montre un profil journalier moyen, durant une durée de 7 mois, de différences de concentrations horaires entre deux stations de référence.

La figure 4A représente les principales étapes d'un procédé de calibration selon l'invention.

La figure 4B montre un exemple d'architecture d'un estimateur de type réseau de neurones.

La figure 5 montre l'effet d'une calibration d'un capteur de gaz selon l'invention.

## EXPOSE DE MODES DE REALISATION PARTICULIERS

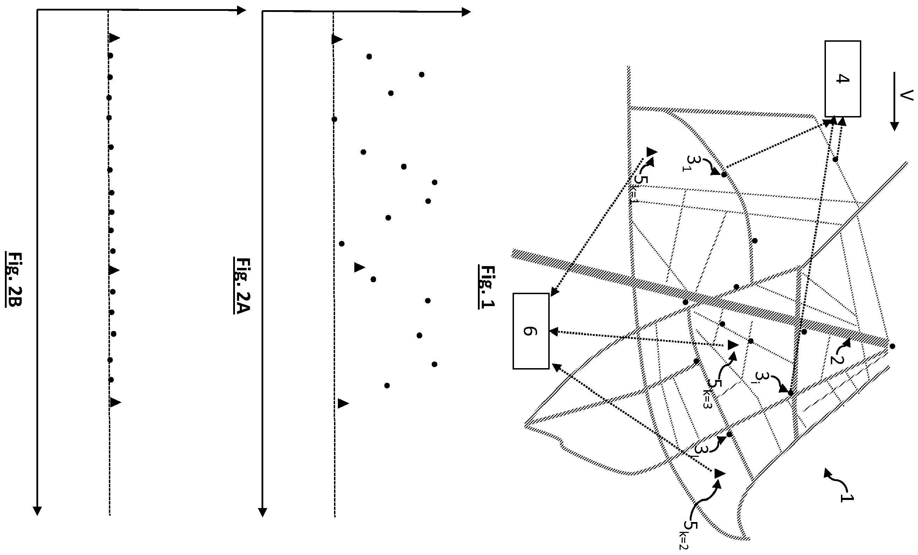

On a représenté, sur la figure 1, une zone géographique dans laquelle on souhaite déterminer une cartographie d'une concentration d'un analyte formant un polluant atmosphérique. L'analyte est par exemple une espèce gazeuse, telle CO, NO, $NO_2$, $O_3$, $SO_2$, $C_6H_6$...Il peut également s'agir de particules fines, par exemple des particules en suspension de type PM10 ou PM 2.5. L'acronyme PM, signifiant Particulate Matter, ou matière particulaire, est connu de l'homme du métier. D'une façon générale, la zone géographique considérée comporte des sources d'émission de l'analyte. Ces sources d'émission peuvent être liées à un trafic de véhicule, à la présence d'installations de chauffage urbain, ou à la présence d'installations industrielles susceptibles d'émettre l'analyte. La zone géographique peut comprendre une zone urbaine ou périurbaine, ou encore une zone industrielle ou aéroportuaire. Dans l'exemple représenté sur la figure 1, la zone géographique 1 est une partie d'une ville, comportant des rues 2 tracées en traits sombres. Des capteurs de gaz $3_1...3_I$ sont répartis dans la zone géographique 1. I est un entier naturel désignant le nombre de capteurs de gaz déployés. Ces capteurs sont par exemple des capteurs de type NDIR, ou des capteurs électrochimiques, optiques, ou à substrat solide, par exemple en oxyde métallique (MOX). Il peut par exemple s'agir de capteurs tels que décrits dans WO2018162848. Sur la figure 1, les capteurs de gaz sont représentés sous la forme de points noirs. Ces capteurs définissent un maillage de la zone géographique considérée. La distance entre deux capteurs adjacents est de préférence inférieure à 1 km, voire inférieure à 500 m. Elle est typiquement de quelques centaines de mètres. La densité des capteurs est typiquement de 5 capteurs par $Km^2$.

Chaque capteur $3_i$, $1 \leq i \leq I$ mesure une concentration d'analyte, par exemple une concentration d'analyte, en différents instants d'une journée. Les mesures sont effectuées à des instants de mesure. Les instants de mesure sont répartis durant le jour et la nuit, généralement selon des intervalles de temps réguliers, par exemple toutes les 1h ou toutes les 2h.

Les capteurs $3_i$ sont reliés à une unité de traitement 4, qui collecte les mesures effectuées à chaque instant de mesure, de façon à établir une cartographie de la concentration en analyte sur la zone géographique considérée, et éventuellement d'effectuer une cartographie prévisionnelle.

La zone géographique 1 comporte également au moins une station de mesure 5, dite station de de référence. Contrairement aux capteurs $3_i$, qui sont des capteurs compacts, et peu onéreux, la station de référence 5 est une station fixe, réalisant des mesures précises durant le jour et la nuit. Généralement, la zone géographique comporte plusieurs stations de référence $5_k$, l'indice k étant un entier compris entre 1 et $N_k$, $N_k$ correspondant au nombre de stations de référence dans la zone géographique considérée. Chaque station de référence $5_k$ est connectée à un réseau de collecte de données 6, ce dernier mettant à disposition les valeurs mesurées. Sur la figure 1, on a représenté trois stations de référence $5_{k=1}$, $5_{k=2}$ et $5_{k=3}$, symbolisées par un triangle. Une station de référence $5_k$ peut par exemple être opérée par un opérateur public, visant à rendre publiques les données relatives à la pollution atmosphérique. Dans l'Union Européenne, le centre de données sur la pollution atmosphérique, opéré par l'Agence Européenne de l'Environnement, assure une mise à disposition de données, dites données ouvertes, mesurées par des stations de mesure fixes. Comme évoqué en lien avec l'art antérieur, de telles stations de référence fournissent des mesures de qualité, mais leur coût et leur encombrement limite leur nombre. Une telle station se présente sous la forme d'un local, doté de dispositifs de prélèvement de l'air ambiant, généralement disposés au niveau du sommet du local. A l'intérieur du local se trouvent des analyseurs spécifiques à certains analytes, tels que ceux préalablement listés.

Un point important de l'invention est d'utiliser les données ouvertes, issues de stations de référence $5_k$, pour effectuer une calibration des capteurs $3_i$.

Les figures 2A et 2B illustrent des concentrations d'un analyte (en l'occurrence $NO_2$) mesurées par des capteurs de gaz $3_i$ et des stations de référence $5_k$ respectivement à un instant de mesure durant la journée ainsi qu'en fin de nuit. Sur ces figures, l'axe des ordonnées représente une concentration d'analyte mesurée, l'axe des abscisses représentant une position de chaque capteur ou station de référence. Les mesures effectuées par un capteur de gaz $3_i$ sont représentées par un point noir. Les mesures issues d'une station de référence $5_k$ sont représentées par un triangle noir. On observe que durant la journée, les mesures réalisées par les capteurs de gaz fluctuent. Durant la nuit, et plus particulièrement en fin de nuit avant la reprise des activités humaines, les mesures tendent à s'uniformiser autour d'un niveau ambiant. Le niveau ambiant est représenté en pointillés sur les figures 2A et 2B. Le niveau ambiant correspond à un niveau de pollution considéré comme uniforme dans la zone géographique étudiée. Il est dû à l'homogénéisation des émissions locales ainsi que de polluants diffusant depuis le voisinage de la zone géographique considérée, un facteur de diffusion étant le vent. L'homogénéisation de la concentration en fin de nuit a été observée pour $NO_2$, mais ce constat est valable pour les polluants liés à l'activité humaine, en particulier au trafic routier ou aérien, au chauffage urbain ou autres activités industrielles cycliques.

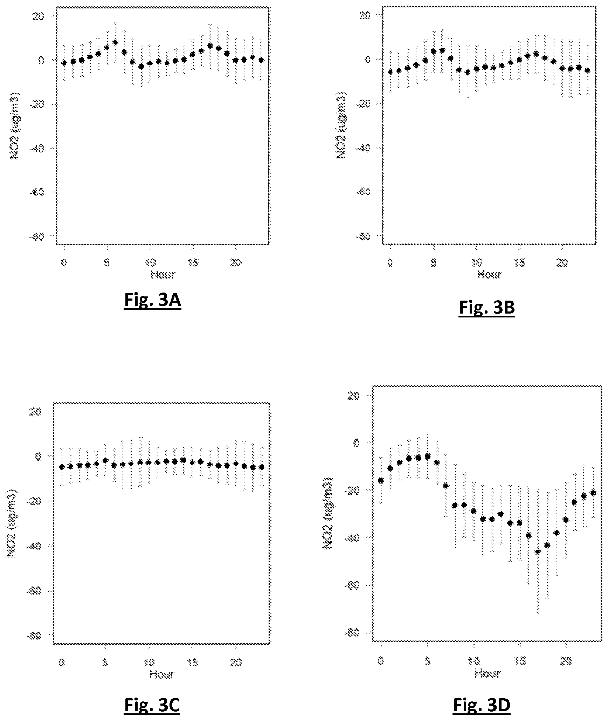

Les stations de référence fixes sont classiquement divisées en des stations de fond, relativement éloignées des principales sources de pollution, et des stations de proximité, disposées à proximité de sources de pollution. Par exemple, la ville de Grenoble dispose d'une dizaine de stations de référence : certaines stations de référence sont considérées comme des stations de fond, d'autres stations de référence sont considérées comme une station de proximité. D'autres stations de référence sont considérées comme des stations intermédiaires, entre une station de proximité et une station de fond. Les figures 3A à 3D représentent une évolution, en fonction de l'heure, des différences moyennes de concentrations de $NO_2$, respectivement mesurées par deux stations de référence différentes. Autrement dit, il s'agit de profils journaliers moyens représentant des différences moyennes de concentrations mesurées, chaque heure, par deux stations de référence différentes. Les moyennes horaires ont été calculées selon une période de 7 mois, s'étendant entre janvier 2018 et juillet 2018. Sur chacune de ces figures, l'axe des abscisses et l'heure (entre 0h et 24h), et l'axe des ordonnées est une concentration de $NO_2$.

La figure 3A et 3B montrent des profils journaliers de différences moyennes des concentrations mesurées par une station intermédiaire et respectivement une première station de fond et une deuxième station de fond. La figure 3C représente le profil journalier des différences moyennes des concentrations mesurées par deux stations de fond. La figure 3D représente un profil journalier des différences moyennes des concentrations mesurées par la station intermédiaire et une station de proximité.

On observe qu'en fin de nuit, c'est-à-dire entre 2h et 6h du matin, ou entre 2h et 5h du matin, sur chacune des figures, les différences des concentrations mesurées sont faibles : leur valeur absolue est inférieure à 10 $\mu g/m^3$. On peut donc conclure que durant cette plage horaire, la concentration en analyte peut raisonnablement être considérée comme uniforme, l'émission de l'analyte étant minimale. Cela confirme que les plages horaires entre 2h et 6h, ou entre 2h et 5h, peuvent être considérées comme des plages temporelles propices à la calibration de capteurs $3_i$ disposés dans le voisinage des stations de référence $5_k$. On utilise le fait que les stations de référence $5_k$ fournissent des données ouvertes, aisément accessibles au public, par le biais du réseau 6. La calibration des capteurs peut donc être réalisée à partir des données publiques transmises par les stations de référence. La plage horaire prise en compte pour la calibration peut correspondre à une plage au cours de laquelle différentes stations de référence mesurent une concentration homogène de l'analyte.

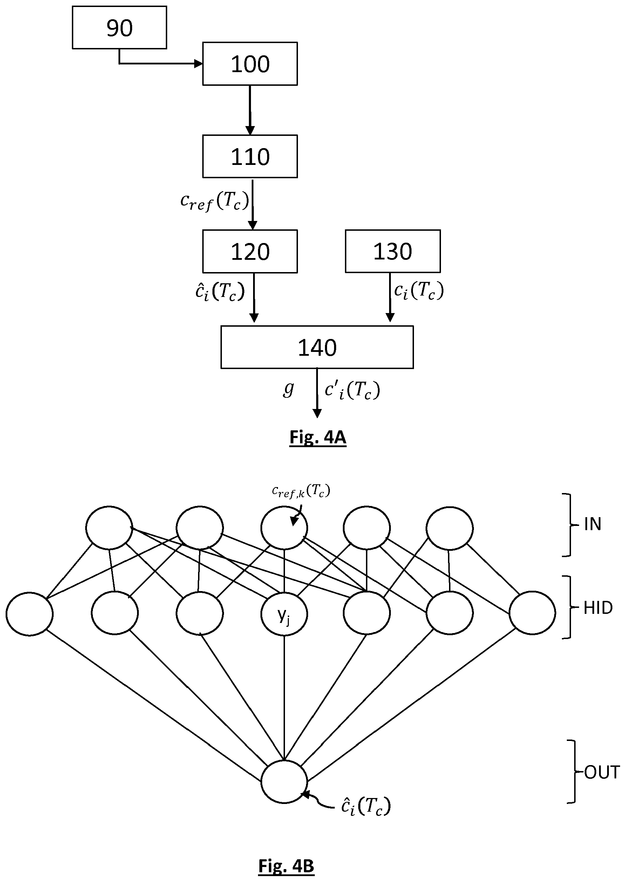

La figure 4A illustre les principales étapes d'un procédé de calibration d'un capteur de gaz $3_i$, selon l'invention.

Etape 100 : association d'un capteur de gaz $3_i$ à une station de référence $5_k$. Lorsque la zone géographique considérée 1 comporte plusieurs stations de référence, on peut sélectionner la station de référence $5_k$ la plus représentative du capteur de gaz. Une telle sélection prend par exemple en compte la distance entre chaque station de référence et le capteur de gaz. La sélection peut également prendre en compte d'autres paramètres de proximité tels que la direction et la vitesse du vent, ou la topologie de la zone géographique considérée, afin de déterminer la station de référence la plus proche compte tenu des paramètres de proximité considérés. Sur la figure 1, on a représenté le vent par un vecteur V.

Selon une variante, le capteur de gaz peut être associé à plusieurs stations de référence, comme décrit par la suite.

La sélection de la station de référence ou des stations de références associées au capteur de gaz peut faire l'objet d'une étape de calibration 90 décrite par la suite.

Etape 110 : prise en compte d'une concentration de référence d'un analyte $c_{ref}(T_c)$ mesurée par la station de référence $5_k$, dans une plage temporelle de calibration. On utilise le fait que la mesure est disponible par le réseau public opérant la station de référence. La plage temporelle de calibration correspond à une plage temporelle au cours de laquelle la variabilité de la concentration d'analyte, dans la zone géographique considérée, est minimale. Ainsi, on considère que la concentration d'analyte, à la position du capteur de gaz, peut être estimée à partir de la mesure effectuée par la station de référence.

Etape 120 : estimation de la concentration d'analyte à la position géographique occupée par le capteur de gaz, à partir de la mesure fournie par la station de référence. Selon une première approche, on considère que la concentration d'analyte à la position occupée par le capteur de gaz est égale à la concentration d'analyte $c_{ref}(T_c)$ mesurée par la station de référence. Cette hypothèse est valide lorsqu'au cours de la plage temporelle de calibration, la répartition de l'analyte est particulièrement homogène. Selon une deuxième approche, la concentration d'analyte à la position occupée par le capteur de gaz est obtenue en appliquant un modèle de propagation à partir de la mesure fournie par la station de référence. Un exemple de modèle de propagation est un modèle de type "Street Canyon", connu de l'homme du métier et usuellement désigné par l'acronyme OPSM (Operational Street Pollution Model).

Ainsi, si $c_{ref}(T_c)$ désigne une concentration d'analyte, résultant de la station de référence, durant la plage temporelle de calibration, et $\hat{c}_i(T_c)$ désigne une concentration de l'analyte estimée à une position i d'un capteur de mesure $3_i$ on a :

$$\hat{c}_i(T_c) = c_{ref}(T_c) \quad (1)$$

ou

$$\hat{c}_i(T_c) = f\left(c_{ref}(T_c)\right) \quad (2)$$

$f$ désignant un modèle de propagation.

Etape 130

Mesure de la concentration d'analyte $c_i(T_c)$ par le capteur de gaz, durant la plage temporelle de calibration. Cette mesure est effectuée à un instant de mesure appartenant à la période temporelle de calibration. Elle n'est pas forcément à l'instant de référence, auquel la concentration de référence $c_{ref}(T_c)$ est mesurée. Toutefois, l'instant de mesure et l'instant de référence appartiennent à la plage temporelle de calibration.

Selon une variante, la concentration de référence $c_{ref}(T_c)$ est établie par une moyenne de concentrations mesurées par la station de référence durant la plage temporelle de calibration. La concentration d'analyte $c_i(T_c)$ peut également résulter d'une moyenne de concentrations mesurées par le capteur de gaz durant la plage temporelle de calibration.

Etape 140 : calibration du capteur de gaz

Au cours de cette étape, on compare la concentration de l'analyte $c_i(T_c)$ mesurée par le capteur $3_i$ durant la plage temporelle de calibration $T_c$, avec la concentration estimée $\hat{c}_i(T_c)$ lors de l'étape 120, et on calibre le capteur de gaz $3_i$ en fonction de la comparaison.

Si $c_i'(T_c)$ désigne la concentration de l'analyte à l'issue de la calibration,

$$c_i'(T_c) = g\left(c_i(T_c),\ \hat{c}_i(T_c)\right) \quad (3)$$

Où g désigne la fonction de calibration, cette dernière dépendant de $c_i(T_c)$ et de $\hat{c}_i(T_c)$. La fonction de calibration g peut être linéaire, de telle sorte que

$$c_i'(T_c) = a + bc_i(T_c) \quad (4),$$

$a$ et $b$ étant des réels.

Du fait de la calibration, la concentration $c_i'(T_c)$ établie postérieurement à la calibration, est présumée plus exacte que la concentration $c_i(T_c)$ mesurée préalablement à la calibration.

Selon un exemple simple, b = 1 et

$$a = c_i(T_c) - mean\left(\hat{c}_i(T_c) - c_i(T_c)\right) \quad (5)$$

où $mean\left(\hat{c}_i(T_c) - c_i(T_c)\right)$ désigne une moyenne de la différence $\hat{c}_i(T_c) - c_i(T_c)$ durant plusieurs plages temporelles de calibration, par exemple des plages de calibration au cours des $n$ derniers jours, $n$ étant par exemple compris entre 2 et 10. Le fait de considérer une moyenne de la différence entre la valeur estimée $\hat{c}_i(T_c)$ et la valeur mesurée $c_i(T_c)$, selon plusieurs plages de calibration, permet de diminuer l'impact du bruit du capteur $3_i$.

La comparaison entre $\hat{c}_i(T_c)$ et $c_i(T_c)$ peut prendre la forme d'une différence, comme indiqué ci-dessus, mais également d'un ratio.

Les étapes 100 à 140 sont effectuées périodiquement, par exemple chaque jour, ou chaque semaine.

Selon un mode de réalisation, la calibration n'est effectuée qu'en présence d'un vent dont la vitesse est supérieure à un seuil prédéterminé. On considère en effet que le vent est un facteur supplémentaire d'homogénéisation de la concentration en analyte avant ou durant la période la plage temporelle de calibration.

Selon une variante du mode de réalisation précédemment décrit, la calibration du capteur de gaz est effectuée à partir de plusieurs stations de mesure de référence. Il peut par exemple s'agir de $K$ stations de référence $5_k$ les plus proches compte tenu de paramètres de proximité tels que la distance et/ou la direction et la vitesse du vent et/ou la topographie de la zone géographique considérée. $K$ est un entier naturel supérieur à 1. L'estimation $\hat{c}_i(T_c)$ peut être effectuée par une moyenne pondérée des mesures de références $c_{ref,k}(T_c)$ résultant des $K$ stations de mesure de référence considérées. L'indice $k$ tel que $1 < k < K$ correspond à une station de référence prise en compte.

Le procédé décrit ci-avant peut comporter une étape d'apprentissage 90, au cours de laquelle on sélectionne la station de référence, ou les stations de référence, dont les mesures de la concentration d'analyte sont les plus corrélées à la concentration d'analyte à la position du capteur de mesure. L'étape d'apprentissage peut également permettre d'estimer la plage temporelle de calibration $T_c$ la plus propice à la réalisation de la calibration. Il s'agit de la plage temporelle lors de laquelle l'estimation $\hat{c}_i(T_c)$ de la concentration d'analyte à la position du capteur est considérée comme la plus fiable. L'étape d'apprentissage peut également permettre de définir l'estimation $\hat{c}_i(T_c)$ de la concentration d'analyte au niveau de la position du capteur de gaz à partir des mesures $c_{ref,k}(T_c)$ respectivement issues de chaque station de référence sélectionnée.

Selon un mode de réalisation, l'étape d'apprentissage est basée sur un algorithme de type machine learning (apprentissage automatique). Il peut notamment s'agir d'un algorithme de type réseau de neurones. Ce type d'architecture, connue de l'homme du métier, comporte une couche d'entrée IN, comportant des données d'entrée, au moins une couche intermédiaire HID, ou couche cachée, et une couche de sortie OUT, comportant la grandeur à estimer, en l'occurrence la concentration d'analyte $\hat{c}_i(T_c)$ au niveau du capteur de gaz. Par exemple, on ne considère qu'une seule couche intermédiaire.

Les données d'entrées, formant la couche d'entrée IN, comprennent les concentrations d'analyte $c_{ref,k}(T_c)$ respectivement mesurées par chaque station de référence $5_k$ considérée. La couche d'entrée IN peut comporter d'autres données d'entrée, par exemple des mesures d'autres analytes, que l'on considère comme corrélés à l'analyte que l'on cherche à quantifier. Les autres mesures peuvent également comprendre des paramètres atmosphériques, choisis par exemple parmi la température, l'humidité, la direction du vent, la vitesse du vent.

La couche intermédiaire HID forme au moins une couche cachée, comportant des nœuds $y_j$, ou neurones. Le nombre de nœuds peut être déterminé de façon arbitraire par l'homme du métier, ou au cours de l'étape d'apprentissage. Le nombre de couches cachées peut également être déterminé au cours de l'étape d'apprentissage. A chaque nœud $y_j$, et pour chaque donnée d'entrée $c_{ref,k}(T_c)$, correspond un facteur de pondération $w_{k,j}$ déterminé au cours de l'étape d'apprentissage. L'architecture du réseau de neurones peut être paramétrée par un algorithme dédié sous l'environnement MATLAB ou l'environnement PYTHON.

L'apprentissage permet de déterminer, entre autres, les facteurs de pondération de la couche cachée. Dans l'exemple considéré, la couche cachée comporte 30 nœuds. Chaque nœud est relié à une donnée d'entrée par un facteur de pondération et un biais.

La figure 4B schématise un exemple d'architecture d'un réseau de neurones comportant 3 couches :

- la couche d'entrée IN, comportant les données d'entrée $c_{ref,k}(T_c)$ ;
- la couche cachée HID, comportant des nœuds (ou neurones) $y_j$, usuellement désignés par le terme anglosaxon "node". L'indice j est un entier compris entre 1 et $N_j$, $N_j$ étant un entier généralement supérieur ou égal à 10, et pouvant dépasser 1000.
- la couche de sortie OUT, comportant l'estimation $\hat{c}_i(T_c)$.

Chaque nœud de la couche intermédiaire est relié à chaque donnée d'entrée. Sur la figure 4B, on n'a pas représenté toutes les liaisons, à des fins de clarté.

L'algorithme est mis en œuvre par l'unité de traitement 4. L'algorithme utilise des données physiques mesurées, correspondant aux les concentrations d'analyte $c_{ref,k}(T_c)$ mentionnées ci-dessus, ainsi que les autres données d'entrée précédemment évoquées.

A chaque nœud $y_j$ est attribué un facteur de pondération $w_{k,j}$ associé à une donnée d'entrée $x_k$. Ainsi, chaque facteur de pondération $w_{k,j}$ est associé à une donnée d'entrée $c_{ref,k}(T_c)$ et à un nœud $y_j$. A chaque nœud est également une valeur de biais $w_{0,j}$. Les facteurs de pondération $w_{k,j}$ ainsi que le biais $w_{0,j}$ de chaque nœud sont déterminés au cours de la calibration. Chaque nœud $y_j$ met en œuvre une fonction d'activation $f_j$, de telle sorte

que :

$$y_j = f_j \left( w_{0,j} + \sum_k w_{k,j}\, c_{ref,k}(T_c) \right) \quad (6)$$

La forme de chaque fonction d'activation $f_j$ est déterminée par l'homme du métier. Il peut par exemple s'agir d'une fonction d'activation $f_j$ est une fonction de type tangente hyperbolique. Les valeurs de chaque nœud $y_j$ sont combinées pour estimer la variable de sortie $\hat{c}_i(T_c)$.

Au cours de l'étape de calibration, les différents paramètres de l'algorithme, en l'occurrence les facteurs de pondération, les biais, et les fonctions d'activation, sont définis sur la base de données de test. Les données de test sont d'une part les valeurs de la concentration d'analyte mesurées au niveau de chaque station de référence, d'autre part la valeur de la concentration d'analyte mesurée au niveau du capteur de gaz, de préférence à l'aide d'un détecteur présentant une précision élevée.

La calibration peut également permettre de sélectionner les stations de référence présentant la meilleure puissance prédictive, c'est-à-dire les $K$ stations de référence dont les mesures sont les plus significativement corrélées à la concentration d'analyte à la position du capteur de gaz. De même, la calibration peut également permettre de déterminer la plage temporelle de calibration $T_c$ la plus appropriée, durant laquelle l'estimation de $\hat{c}_i(T_c)$ est la plus exacte.

Selon un modèle plus raffiné, la couche de sortie peut comporter une estimation d'une quantité d'analyte $\hat{c}_i(T_c)$ à différentes positions géographiques, chaque position correspondant à un capteur de gaz différent. La couche de sortie comporte alors autant de données que de capteurs de gaz à calibrer.

A l'issue de l'étape d'apprentissage, les étapes 100 à 140 précédemment décrites sont mises en œuvre :

- au cours de l'étape 100, le capteur de gaz $3_i$ est associé aux stations de références $5_k$ sélectionnées lors de l'apprentissage ;
- au cours de l'étape 110, on prend en compte les concentrations de références $c_{ref,k}(T_c)$ mesurées par les $K$ stations de référence sélectionnées, ainsi que les éventuelles autres données d'entrée préalablement décrites ;
- au cours l'étape 120, on met en œuvre le réseau de neurones pour estimer la concentration d'analyte $\hat{c}_i(T_c)$ à la position du capteur de gaz.
- les étapes 130 et 140 sont similaires à celles préalablement décrites.

Essai

Des essais ont été réalisés dans la ville de Grenoble, comportant les stations de référence précédemment décrites. Durant la phase d'essais, on a disposé un capteur de gaz à la même position que la station de référence intermédiaire. Dans cet exemple, l'analyte considéré est l'ozone ($O_3$). Le capteur de gaz a été calibré chaque jour en mettant en œuvre les étapes décrites ci-avant, la plage temporelle de calibration étant comprise entre 3 heures et 5 heures du matin. L'essai s'est déroulé entre le 20 juin 2018 et le 31 juillet 2018. La calibration a été réalisée en mettant en œuvre l'équation (5).

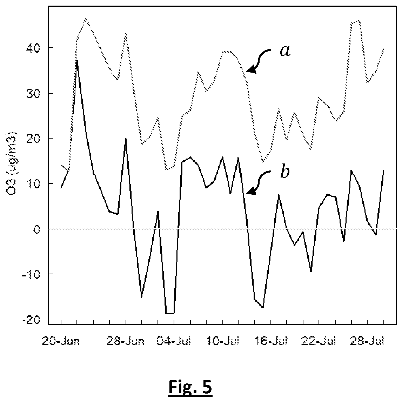

On a représenté, sur la figure 5, l'évolution temporelle de la différence entre les moyennes journalières fournies par la station de référence et celles résultant du capteur de gaz :

- sans prendre en compte la calibration (courbe *a*), c'est-à-dire en se basant uniquement sur une calibration initiale effectuée avant l'installation du capteur de gaz;
- en appliquant la calibration chaque jour (courbe *b*).

Durant les premiers jours, les courbes *a* et *b* sont presque confondues. Cependant, après quelques jours, les courbes *a* et *b* se distinguent nettement l'une de l'autre, sous l'effet de la calibration régulière du capteur de gaz. Les mesures obtenues par la station de référence sont considérées comme exactes.

On a calculé une moyenne des valeurs représentées sur chaque courbe, le moyenne étant de 29.13 $\mu$g/m$^3$ pour la courbe *a* et de 4.56 $\mu$g/m$^3$ pour la courbe *b*. Cela montre qu'en procédant à des calibrations régulières du capteur, les concentrations mesurées par ce dernier sont plus représentatives de la réalité.

L'invention permet d'effectuer une calibration astucieuse d'un capteur de gaz, en utilisant des données disponibles, résultant de stations de référence réparties sur le territoire. La calibration est effectuée à distance, sur la base de mesures résultant du capteur de gaz et d'une estimation d'une concentration d'analyte à la position occupée par le capteur de gaz. La calibration ne nécessite pas d'intervention physique sur le capteur de gaz, et ne nécessite pas une exposition de ce dernier à un gaz étalon. La calibration étant particulièrement simple, elle peut être réalisée fréquemment, par exemple chaque jour ou chaque semaine.

L'invention pourra être utilisée pour calibrer des capteurs de gaz répartis dans un environnement urbain ou périurbain, ou le long d'un axe routier, ou d'un aéroport, ou encore dans le voisinage d'une installation industrielle.

**Revendications**

1. Procédé de calibration d'un capteur de gaz ($3_i$), le capteur de gaz appartenant à un réseau de capteurs ($3_1...3_I$) répartis en différentes positions dans une zone géographique, le capteur de gaz étant destiné à mesurer une concentration d'un analyte dans l'air à différents instants de mesure, la zone géographique comportant au moins une station de référence ($5_K$), distante du capteur de gaz, la station de référence étant destinée à mesurer, à différents instants de référence, une concentration d'analyte dans l'air, le procédé comportant les étapes suivantes :

    a) association d'au moins une station de référence au capteur de gaz;
    b) durant une plage temporelle de calibration ($T_c$), mesure d'une concentration d'analyte ($c_i(T_c)$) par le capteur de gaz et prise en compte d'une concentration d'analyte ($c_{ref}(T_c)$) mesurée par chaque station de référence associée au capteur de gaz ;
    c) à partir de la mesure de la concentration d'analyte mesurée par chaque station de référence lors de la plage temporelle de calibration, estimation d'une concentration d'analyte à la position du capteur de gaz;
    d) comparaison entre la concentration d'analyte ($\hat{c}_i(T_c)$) estimée lors de l'étape c) et la concentration d'analyte ($c_i(T_c)$) mesurée par le capteur de gaz lors de l'étape b) ;
    e) en fonction de la comparaison effectuée lors de l'étape d), calibration du capteur de gaz ;

    • l'analyte étant émis, dans la zone géographique, selon une concentration variable au cours d'une journée, la concentration d'analyte émise variant entre un minimum et un maximum au cours de chaque journée; le procédé étant **caractérisé en ce que**:
    • la plage temporelle de calibration ($T_c$) est déterminée de façon à correspondre à une émission minimale de l'analyte dans la zone géographique considérée, durant chaque journée.

2. Procédé selon la revendication 1 comportant, préalablement aux étapes a) à e), une étape d'apprentissage de façon à :

    - sélectionner une station de référence ou plusieurs stations de référence, parmi une pluralité de stations de référence, de telle sorte que durant la plage temporelle de calibration, la concentration d'analyte mesurée par chaque station de référence sélectionnée est corrélée à la concentration d'analyte à la position du capteur de gaz;
    - déterminer un estimateur de la concentration d'analyte à la position du capteur de gaz à partir de la concentration d'analyte mesurée par chaque station de référence sélectionnée ;

    de telle sorte que:

    - lors de l'étape a), chaque station de référence sélectionnée lors de l'étape d'apprentissage est associée au capteur de gaz ;
- lors de l'étape c), l'estimation de la concentration d'analyte à la position du capteur de gaz est effectuée en appliquant l'estimateur déterminé lors de l'étape d'apprentissage.

3. Procédé selon la revendication 2, dans lequel la plage temporelle de calibration est déterminée lors de l'étape d'apprentissage.

4. Procédé selon l'une quelconque des revendications 2 ou 3, dans lequel l'étape d'apprentissage met en œuvre un réseau de neurones, de façon à sélectionner au moins une station de référence et établir l'estimateur et éventuellement la plage temporelle de calibration.

5. Procédé selon l'une quelconque des revendications précédentes, dans lequel lors de l'étape c), la concentration d'analyte à la position du capteur de gaz est :

- considérée comme égale à la concentration d'analyte ($c_{ref}(T_c)$) mesurée par une station de référence associée au capteur de gaz ;
- ou estimée en appliquant un modèle de propagation (*f*), à partir de la concentration d'analyte ($c_{ref}(T_c)$) mesurée par au moins une station de référence associée au capteur de gaz.

6. Procédé selon l'une quelconque des revendications précédentes, dans lequel l'analyte est émis par des moyens de transport ou de chauffage, la plage temporelle de calibration étant comprise entre minuit et 6 heures du matin, et de préférence entre 3 heures du matin et 6 heures du matin

7. Procédé selon l'une quelconque des revendications précédente, dans lequel :

- l'aire géographique comporte plusieurs stations de référence ;
- l'étape a) comporte une association d'une station de référence en fonction d'une distance entre le capteur de gaz et chaque station de référence.

8. Procédé selon la revendication 7,
dans lequel l'étape a comporte une association d'une station de référence en fonction de la vitesse d'un vent se propageant dans la zone géographique, et/ou en fonction de la direction du vent se propageant dans la zone géographique.

9. Procédé selon l'une quelconque des revendications précédentes, dans lequel l'analyte est de type : NO ou $NO_2$ ou $O_3$ ou $SO_2$ ou CO ou $C_6H_6$ ou matière particulaire.

10. Procédé selon l'une quelconque des revendications précédentes, dans lequel lors de l'étape e, la calibration du capteur est effectuée en mettant en œuvre une fonction de calibration, appliquée à la comparaison des mesures de calibration effectuée lors de l'étape d).

11. Procédé selon l'une quelconque des revendications précédentes, dans lequel chaque station de référence est connectée à une base de données publique, de telle sorte que lors de l'étape b), la concentration d'analyte mesurée par la station de référence est issue de la base de données publique

12. Dispositif d'estimation d'une concentration d'un analyte dans une zone géographique, le dispositif comportant :

• plusieurs capteurs de gaz ($3_1...3_I$) répartis dans la zone géographique, chaque capteur de gaz étant configuré pour mesurer une concentration d'analyte à différents instants de mesure,
• une unité de traitement (4), recevant les mesures collectées au niveau de la pluralité de capteurs de gaz ($3_1...3_I$);
• le dispositif étant **caractérisé en ce que** l'unité de traitement est configurée pour mettre en œuvre un procédé selon l'une quelconque des revendications 1 à 11, en utilisant au moins une station de mesure de référence ($5_k$) située dans la zone géographique.

**Patentansprüche**

1. Verfahren zur Kalibrierung eines Gassensors ($3i$), wobei der Gassensor zu einem Netz aus Sensoren ($3_1...3_I$) gehört, die in verschiedenen Positionen in einem geografischen Bereich verteilt sind, wobei der Gassensor dazu bestimmt ist, eine Konzentration eines Analyten in der Luft zu verschiedenen Messzeitpunkten zu messen, wobei der geografische Bereich wenigstens eine Referenzstation ($5_k$) aufweist, die vom Gassensor entfernt ist, wobei die Referenzstation dazu bestimmt ist, zu verschiedenen Referenzzeitpunkten eine Analytkonzentration in der Luft zu messen, wobei das Verfahren die folgenden Schritte aufweist:

a) Zuordnen wenigstens einer Referenzstation zum Gassensor;
b) während eines Kalibrierungszeitbereichs ($T_c$), Messen einer Analytkonzentration ($c_i(T_c)$) durch den Gassensor und Berücksichtigen einer Analytkonzentration ($c_{ref}(T_c)$), die von jeder Referenzstation gemessen wird, die dem Gassensor zugeordnet ist;
c) anhand der Messung der Analytkonzentration, die von jeder Referenzstation im Kalibrierungszeitbereich gemessen wird, Schätzen einer Analytkonzentration an der Position des Gassensors;

d) Vergleich zwischen der Analytkonzentration ($\hat{c}_i(T_c)$), die beim Schritt c) geschätzt wird, und der Analytkonzentration ($c_i(T_c)$), die vom Gassensor beim Schritt b) gemessen wird;

e) in Abhängigkeit von dem Vergleich, der beim Schritt d) durchgeführt wird, Kalibrieren des Gassensors;

    • wobei der Analyt im geografischen Bereich in einer Konzentration emittiert wird, die im Verlauf eines Tages variabel ist, wobei die emittierte Analytkonzentration im Verlauf jedes Tages zwischen einem Minimum und einem Maximum variiert;

wobei das Verfahren **dadurch gekennzeichnet ist, dass**:

    • der Kalibrierungszeitbereich ($T_c$) so bestimmt wird, dass er einer Minimalemission des Analyten im betrachteten geografischen Bereich während jedes Tages entspricht.

2. Verfahren nach Anspruch 1, aufweisend vor den Schritten a) bis e) einen Schritt des Lernens zum:

    - Auswählen einer Referenzstation oder mehrerer Referenzstationen aus einer Mehrzahl von Referenzstationen, so dass während des Kalibrierungszeitbereichs die Analytkonzentration, die von jeder ausgewählten Referenzstation gemessen wird, mit der Analytkonzentration an der Position des Gassensors korreliert;

    - Bestimmen eines Schätzers der Analytkonzentration an der Position des Gassensors anhand der Analytkonzentration, die von jeder ausgewählten Referenzstation gemessen wird;

so dass:

    - beim Schritt a) jede Referenzstation, die beim Schritt des Lernens ausgewählt wird, dem Gassensor zugeordnet wird;

    - beim Schritt c) das Schätzen der Analytkonzentration an der Position des Gassensors unter Anwendung des Schätzers durchgeführt wird, der beim Schritt des Lernens bestimmt wird.

3. Verfahren nach Anspruch 2, wobei der Kalibrierungszeitbereich beim Schritt des Lernens bestimmt wird.

4. Verfahren nach einem der Ansprüche 2 oder 3, wobei beim Schritt des Lernens ein neuronales Netz verwendet wird, um wenigstens eine Referenzstation auszuwählen und den Schätzer und schließlich den Kalibrierungszeitbereich zu erstellen.

5. Verfahren nach einem der vorhergehenden Ansprüche, wobei beim Schritt c) die Analytkonzentration an der Position des Gassensors:

    - als gleich der Analytkonzentration ($c_{ref}(T_c)$) erachtet wird, die von einer Referenzstation gemessen wird, die dem Gassensor zugeordnet ist;

    - oder unter Anwendung eines Ausbreitungsmodells (f) anhand der Analytkonzentration ($c_{ref}(T_c)$) geschätzt wird, die von wenigstens einer Referenzstation gemessen wird, die dem Gassensor zugeordnet ist.

6. Verfahren nach einem der vorhergehenden Ansprüche, wobei der Analyt von Transport- oder Heizmitteln emittiert wird, wobei der Kalibrierungszeitbereich zwischen Mitternacht und 6 Uhr morgens und vorzugsweise zwischen 3 Uhr morgens und 6 Uhr morgens liegt.

7. Verfahren nach einem der vorhergehenden Ansprüche, wobei:

    - die geografische Fläche mehrere Referenzstationen aufweist;

    - der Schritt a) ein Zuordnen einer Referenzstation je nach einer Entfernung zwischen dem Gassensor und jeder Referenzstation aufweist.

8. Verfahren nach Anspruch 7, wobei der Schritt a ein Zuordnen einer Referenzstation je nach der Geschwindigkeit eines Winds, der sich im geografischen Bereich ausbreitet, und/oder je nach der Richtung des Winds, der sich im geografischen Bereich ausbreitet, aufweist.

9. Verfahren nach einem der vorhergehenden Ansprüche, wobei der Analyt vom folgenden Typ ist: NO oder $NO_2$ oder $O_3$ oder $SO_2$ oder CO oder $C_6H_6$ oder partikelförmiges Material.

10. Verfahren nach einem der vorhergehenden Ansprüche, wobei beim Schritt e die Kalibrierung des Sensors unter Verwendung einer Kalibrierungsfunktion durchgeführt wird, die auf den Vergleich der Kalibrierungsmessungen angewandt wird, der beim Schritt d) durchgeführt wird.

11. Verfahren nach einem der vorhergehenden Ansprüche, wobei jede Referenzstation an eine öffentliche Datenbank angebunden ist, so dass beim Schritt b) die Analytkonzentration, die von der Referenzstation gemessen wird, von der öffentlichen Datenbank stammt.

**12.** Vorrichtung zur Schätzung einer Konzentration eines Analyts in einem geografischen Bereich, die Vorrichtung aufweisend:

• mehrere Gassensoren ($3_1$...$3_l$), die im geografischen Bereich verteilt sind, wobei jeder Gassensor dazu ausgebildet ist, eine Analytkonzentration zu verschiedenen Messzeitpunkten zu messen,
• eine Verarbeitungseinheit (4), die die Messungen empfängt, die an der Mehrzahl von Gassensoren ($3_1$....$3_l$) erfasst werden;
• wobei die Vorrichtung **dadurch gekennzeichnet ist, dass** die Verarbeitungseinheit dazu ausgebildet ist, unter Verwendung wenigstens einer Referenzmessstation ($5_k$), die sich im geografischen Bereich befindet, ein Verfahren nach einem der Ansprüche 1 bis 11 umzusetzen.

**Claims**

**1.** Method for calibrating a gas sensor ($3_i$), the gas sensor belonging to a network of sensors ($3_1$... $3_l$) distributed between various positions in a geographical region, the gas sensor being intended to measure a concentration of an analyte in the air at various measurement times, the geographical region comprising at least one reference station ($5_k$), which is remote from the gas sensor, the reference station being intended to measure, at various reference times, a concentration of analyte in the air, the method comprising the following steps:

a) associating at least one reference station with the gas sensor;
b) during a calibration time slot ($T_c$), measuring an analyte concentration ($c_i(T_c)$) with the gas sensor and taking into account an analyte concentration ($c_{ref}(T_c)$) measured by each reference station associated with the gas sensor;
c) from the measurement of the analyte concentration measured by each reference station in the calibration time slot, estimating an analyte concentration in the position of the gas sensor;
d) comparing the analyte concentration ($\hat{c}_i(T_c)$) estimated in step c) and the analyte concentration ($c_i(T_c)$) measured by the gas sensor in step b);
e) depending on the comparison made in step d), calibrating the gas sensor;

• the analyte being emitted, in the geographical region, at a concentration that varies over the course of a day, the emitted analyte concentration varying between a minimum and a maximum over the course of each day;

the method being **characterized in that**:

• the calibration time slot ($T_c$) is determined so as to correspond to a minimum emission of the analyte in the geographical region in question, during each day.

**2.** Method according to Claim 1, comprising, prior to steps a) to e), a training step so as to:

- select a reference station or a plurality of reference stations, from among a plurality of reference stations, such that, during the calibration time slot, the analyte concentration measured by each selected reference station is correlated with the analyte concentration in the position of the gas sensor;
- determine an estimator of the analyte concentration in the position of the gas sensor from the analyte concentration measured by each selected reference station;

such that:

- in step a), each reference station selected in the training step is associated with the gas sensor;
- in step c), the analyte concentration in the position of the gas sensor is estimated by applying the estimator determined in the training step.

**3.** Method according to Claim 2, wherein the calibration time slot is determined in the training step.

**4.** Method according to either one of Claims 2 and 3, wherein the training step employs a neural network, so as to select at least one reference station and establish the estimator and possibly the calibration time slot.

**5.** Method according to any one of the preceding claims, wherein, in step c), the analyte concentration in the position of the gas sensor is:

- considered to be equal to the analyte concentration ($c_{ref}(T_c)$) measured by a reference station associated with the gas sensor;
- or estimated by applying a dispersion model ($f$), based on the analyte concentration ($c_{ref}(T_c)$) measured by at least one reference station associated with the gas sensor.

**6.** Method according to any one of the preceding claims, wherein the analyte is emitted by means of transport or heating, the calibration time slot being comprised between midnight and 6 o'clock in the morning, and preferably between 3 o'clock in the morning and 6 o'clock in the morning.

7. Method according to any one of the preceding claims, wherein:

   - the geographical area comprises a plurality of reference stations;
   - step a) comprises an association of a reference station depending on a distance between the gas sensor and each reference station.

8. Method according to Claim 7, wherein step a comprises an association of a reference station depending on the speed of a wind propagating through the geographical region, and/or depending on the direction of the wind propagating through the geographical region.

9. Method according to any one of the preceding claims, wherein the analyte is: NO or $NO_2$ or $O_3$ or $SO_2$ or CO or $C_6H_6$ or particulate matter.

10. Method according to any one of the preceding claims, wherein, in step e, the sensor is calibrated by implementing a calibration function, applied to the comparison of the calibration measurements carried out in step d).

11. Method according to any one of the preceding claims, wherein each reference station is connected to a public database, such that, in step b), the analyte concentration measured by the reference station is taken from the public database.

12. Device for estimating a concentration of an analyte in a geographical region, the device comprising:

   • a plurality of gas sensors ($3_1$... $3_l$) distributed over the geographical region, each gas sensor being configured to measure an analyte concentration at various measurement times,
   • a processing unit (4), which receives the measurements collected by the plurality of gas sen-sors ($3_1$... $3_l$);
   • the device being **characterized in that** the processing unit is configured to implement a method according to any one of Claims 1 to 11, using at least one reference measurement sta-tion ($5_k$) located in the geographical region.

**Fig. 1**

**Fig. 2A**

**Fig. 2B**

**Fig. 3A**

**Fig. 3B**

**Fig. 3C**

**Fig. 3D**

**Fig. 4A**

**Fig. 4B**

**Fig. 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018178561 A
- US 2018136180 A1
- US 2018156766 A1
- WO 2018162848 A